# EUROPEAN PATENT SPECIFICATION

(11) **EP 2 608 774 B1**
(45) Date of publication and mention of the grant of the patent: **12.10.2016**
(21) Application number: 11754333.0
(22) Date of filing: 24.08.2011
(51) Int. Cl.: A61K 9/16, A61K 39/385, A61K 47/26

(54) **POROUS DEGRADABLE POLYELECTROLYTE MICROSPHERES AS VACCINE VECTOR**
PORÖSE ABBAUBARE POLYELEKTROLYT-MIKROKÜGELCHEN ALS IMPFSTOFFVEKTOR
MICROSPHÈRES DÉGRADABLES ET POREUSES À BASE DE POLYELECTROLYTES COMME VECTEURS DE VACCINS

(30) Priority: 24.08.2010 GB 201014121
(43) Date of publication of application: 03.07.2013
(73) Proprietor: Universiteit Gent, 9000 Gent (BE)
(72) Inventor: REMON, Jean Paul, B-9090 Melle (BE); DE GEEST, Bruno, B-9840 De Pinte (BE); DE KOKER, Stefaan, B-9000 Gent (BE); GROOTEN, Johan, B-9920 Lovendegem (BE); VERVAET, Chris, B-8870 Kachtem (BE)
(74) Representative: Gevers Patents
(86) International application number: PCT/EP2011/064507
(87) International publication number: WO 2012/025551

(56) References cited:
- DE KOKER, S. ET AL.: "Biodegradable Polyelectrolyte Microcapsules: Antigen Delivery Tools with Th17 Skewing Activity after Pulmonary Delivery", J. IMMUNOL., 2009, pages 203-211, XP002665087, cited in the application

## Description

### Filed of the invention

The present invention is in the fields of pharmaceutical technology, molecular biology, and immunology.

### Background of the invention

Development of biologic drugs has rapidly expanded in the last decades with the advent of molecular biology techniques. Synthetic vaccines designed to prime the adaptive immune system are sought for a broad range of diseases, including infectious diseases and cancer in both prophylactic and therapeutic settings.

Priming of the adaptive immune system requires inducing both T cell and B cell responses, which are triggered by two different pathways. T cell activation occurs when a cellular intermediate, the dendritic cell, internalizes a protein, proteolyses this antigen into short peptides, and presents these peptides to T cells in the cleft of their major histocompatibility complex (MHC) molecules. B cells, on the other hand, can directly bind protein antigens via-their B cell receptors (membrane-tethered IgM or IgG molecules).

Simultaneously priming cellular and humoral responses requires a dual release of antigen: immediate release where a fraction is freed because it is not bound to the polyelectrolyte, and slow release, where a fraction is bound.

The design of synthetic vaccines that potently stimulate both arms of adaptive immunity remains a significant challenge. In particular, the failure of current vaccination strategies to elicit cellular immune responses, especially CD8+ cytotoxic T cells (CTLs) that can recognize and eliminate infected or malignant cells, is considered to be one of the major impediments.

Dendritic cells and macrophages have the unique capability to present exogenous (extracellular) proteins on MHC class I molecules by a process called cross-presentation [1,2]. Cross-presentation is not efficient for protein antigens when administered in soluble form, and is typically observed only when high concentrations of exogenous protein are used. In contrast, it has been shown that whole intact microbes (or purified protein antigens adsorbed onto latex particles) taken up by phagocytosis can elicit cross-presentation of the delivered antigen very efficiently; these antigens are then processed and presented on MHC class I molecules to naive CD8+ T cells [1,3,4]. As such, the recognition of the evolutionarily engineered response of the immune system to encapsulated microbes or particulate antigens has led to promising approaches into biologics drug delivery systems.

Polymeric multilayer capsules [5-10] have been concocted-as carriers for the delivery of antigens to dendritic cells. These capsules are made by alternate deposition of polymers (the so-called Layer-by-Layer technology or LbL), [11,12] either through electrostatic interaction [13-15] or hydrogen bonding [16] onto a sacrificial template followed by the decomposition of the sacrificial template resulting in hollow capsules, thereby allowing an efficient antigen encapsulation under non-denaturing conditions.

Some papers [17-24] have demonstrated the potential of polyelectrolyte microcapsules as antigen delivery vehicles to dendritic cells. Although these particulate carriers have the capacity to evoke potent cellular immunity, their clinical and industrial applications have still been hindered by practical problems deriving from their production. A major drawback is the low antigen encapsulation efficiency whereas maximizing antigen loading in carriers is highly desired in order to elicit robust immunity with reasonable vaccine doses. Another disadvantage is the use of chemical solvents and physical stresses that negatively affect antigen stability, increasing the risk of denaturation or destruction of protein antigens during synthesis and storage within the carrier. It is worthwhile to remind that most proteins and antigens are labile to high processing temperatures. Another key factor is the need for carriers with increased porosity to allow protease infiltration into the microcapsules and subsequent proteolytic processing. The involvement of complex and labor intensive multi-step processes -to generate these particulate carriers- makes them not interesting either for industrial application. Minimizing the use of toxic or pharmaceutically non-acceptable chemicals or treatments during the preparation of these particulate carriers also accounts for a major burden for later clinical applications.

Spray-drying is a widely used technique in the pharmaceutical industry for compounding drugs in particle form. However, when the generated particles are mixed in water, for example for administration by injection to the body, their typical behavior is either to aggregate creating clumps, or to dissolve, leaving the most of the drug in soluble form, which phenomena are not desired in the present case. In addition, it is not predictable whether the amorphous or crystal states of the particle components remain the same after spray-drying. Such state forms have tremendous consequences in terms of particle stability, particularly when processed as a suspension. Importantly, for biologic drug formulations, polyols are preferably used in amorphous state since they impart higher protein stability than the crystalline or hydrate forms thereof.

Against these prejudices and requirements, the inventors have surprisingly found that when spray-drying an aqueous mixture of a polyol and a polyelectrolyte complex, the polyol transits to its amorphous form. This phenomenon has not been observed when the polyol was spray-dried alone, or when a mixture of a polyol and a polyelectrolyte complex was freeze-dried.

Also remarkably, the inventors have surprisingly found that when spray-drying an aqueous mixture of a polyol, a polyelectrolyte complex, and a biologic drug, the obtained solid particles remain stable when re-constituted in a physiological solution for parenteral administration. More advantageously, the spray-drying process conveys enhanced encapsulation efficiency when compared to the particles prepared by the LbL technology. Even more advantageously, the amorphous polyol within the spray-dried obtained solid particles imparts improved stability to the biologic drug. Even more remarkably, the inventors demonstrate that by varying the particle composition it is possible to obtain a dual drug release. Upon redispersion of the dry powder (i.e. spray dried polyelectrolyte microspheres) a fraction of the drug is retained within the polyelectrolyte framework (i.e. remains stably encapsulated within the microspheres) while another fraction is immediately released into the surrounding medium. This feature is of importance for the induction of humoral (antibody mediated) immune responses as for this purpose intact drug should reach B-cells, either via passive drainage through the lymphatics or transported via dendritic cells (DCs). Since the drug encapsulated within polyelectrolyte microspheres is likely to be processed (i.e. cleaved into peptide fragments) once internalized by DCs, it is unlikely that DCs will pass intact drug (that was internalized under the form of polyelectrolyte microspheres) to B-cells. Therefore, a formulation that contains both encapsulated and soluble drug should be capable to target, besides DCs with encapsulated drug, also B cells with intact soluble drug.

EP2135601 (Capsulution Nanoscience AG) relates to drug formulations for the stabilization of amorphous forms of drugs. In particular it relates to pharmaceutical compositions comprising sponge-like carrier matrices, particularly polyelectrolyte complexes or porous particles. The teachings of EP2135601 do not disclose the advantageous amorphous polyol, neither the dual release spherical formulations with enhancing immunogenic properties of the present invention.

It is an object of the present invention the provision of a drug delivery system for biologic drugs.

It is an object of the present invention the provision of a drug delivery system for biologic drugs with a controlled dual release.

It is an object of the present invention the provision of a biologic drug delivery system that can deliver biologic drugs extracellularly.

It is an object of the present invention the provision of a biologic drug delivery system that can deliver biologic drugs to phagocytes, especially dendritic cells.

It is an object of the present invention the provision of a biologic drug delivery system that can elicit a cellular immune response.

It is an object of the present invention the provision of a biologic drug delivery system that can enhance immunogenic response.

It is an object of the present invention the provision of a biologic drug delivery system that can enhance antigen presentation especially that can promote MHC-I mediated cross-presentation to CD8+ T cells.

It is an object of the present invention the provision of a biologic drug delivery system that can protect a biologic drug from degradation before reaching dendritic cells.

It is an object of the present invention the provision of a biologic drug delivery system with a suitable bioavailability.

It is an object of the present invention the provision of a biologic drug delivery system with optimal chemical and physical stability. In particular, it is an object of the present invention the provision of a biologic drug delivery system that remains stable after extracting the amorphous polyol (acting a sacrificial component), without a significant leakage of the biologic drug. It is an object of the present invention the provision of a biologic drug delivery system that remains stable after re-constitution with a physiological solution. It is also an object of the present invention the provision of a biologic drug delivery system with improved biologic drug stability.

It is an object of the present invention the provision of a biologic drug delivery system with enhanced encapsulation efficiency, thereby minimizing the waste of expensive drug and polyelectrolytes.

It is an object of the present invention the provision of a biologic drug delivery system that can be prepared in a one- or two-step method, avoiding complex and labor intensive multi-step processes, thereby facilitating large scale production.

It is an object of the present invention the provision of a biologic drug delivery system that avoids, for its preparation, the electrostatic layer-by-layer (ELbL) assembly method, which is a labor intensive multi-step process.

It is an object of the present invention the provision of a biologic drug delivery system that can be prepared without using, or with a minimal use of, organic solvents.

It is an object of the present invention the provision of a biologic drug delivery system whose preparation is environment-friendly.

It is an object of the present invention the provision of a biologic drug delivery system that comprises pharmaceutically acceptable excipients, with minimal or acceptable toxicity, or with low immunogenicity.

It is an object of the present invention the provision of a biologic drug delivery system that comprises pharmaceutically acceptable excipients that are versatile, such as the polyelectrolyte complex, whose in vivo degradation rate can be modulated by the structural alteration thereof.

It is an object of the present invention the provision of a biologic drug delivery system that avoids non-polymeric chemical cross-linkers.

It is an object of the present invention the provision of a biologic drug delivery system in particle form that presents an increased electrostatic potential of the particles, which is interesting for an optimal suspension stability.

It is an object of the present invention the provision of a biologic drug delivery system that presents an increased surface area, being more effective in capturing more drug per mg of sacrificial component, than the processes of the prior art consisting of i) physical adsorption-adsorption of drug onto preformed sacrificial component, or ii) coprecipitation-drug capture by sacrificial microparticles, each i) and ii) followed by polyelectrolyte LbL assembly. This increased surface area allows for an increased drug load and loading efficiency, and it is particularly achieved by the microparticles obtainable by the spray-drying method, which present a highly dense network of components and nanosized pores formed after extraction of the sacrificial component, i.e. the amorphous polyol.

### Summary of the invention

The present invention discloses a composition comprising freeze-dried microspheres comprising a polyelectrolyte complex, an antigen and a polyol, characterised in that said polyelectrolyte complex is a complex of dextran sulphate and poly-L-arginine and said polyol is mannitol, and wherein said mannitol is in amorphous form.

In one embodiment disclosing any one of the compositions of the present invention, said composition is in a particle form, or a powder.

The present invention further discloses a suspension comprising the composition according to any one of the embodiments presented herein.

In one embodiment said suspension exhibits a dual drug release.

The present invention further discloses a pharmaceutical composition comprising the composition according to any one of the embodiments of the present invention and a pharmaceutical acceptable carrier, wherein the pharmaceutical composition comprises a pharmaceutically effective amount of one or more antigens.

The present invention further discloses a method for the preparation of the freeze-dried microspheres according to any one of the embodiments presented herein, said method comprising:
a) preparing an aqueous feed comprising mannitol, a polyelectrolyte complex of dextran sulphate and poly-L-arginine, and one or more antigens, at a total solid concentration of about 0.1 to 10%;
b) spray-drying the aqueous feed prepared in step a); and
c) optionally extracting mannitol with a suitable complexing agent.

### Brief description of the figures

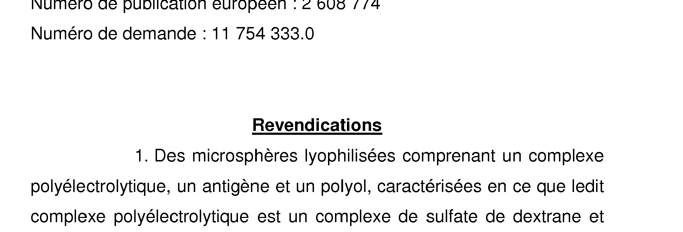

Figure 1. Schematic illustration of the encapsulation of antigen (triangles) into porous polyelectrolyte (curves) microspheres by spray-drying a mixture of antigen, polyelectrolytes and mannitol (discs) followed by extraction of the mannitol by redispersion in water.
Figure 2. Scanning electron microscopy images at different zoom of spray-dried mannitol/DS/OVA/P_{L}ARG microspheres (A) before and (B) after extraction of the mannitol with water.
Figure 3. Confocal microscopy images of spray-dried solid mannitol/DS/OVA/P_{L}ARG microspheres using green fluorescent OVA. (A) fluorescence channel, (B) transmission channel and (C) overlay between green fluorescence and transmission channel.

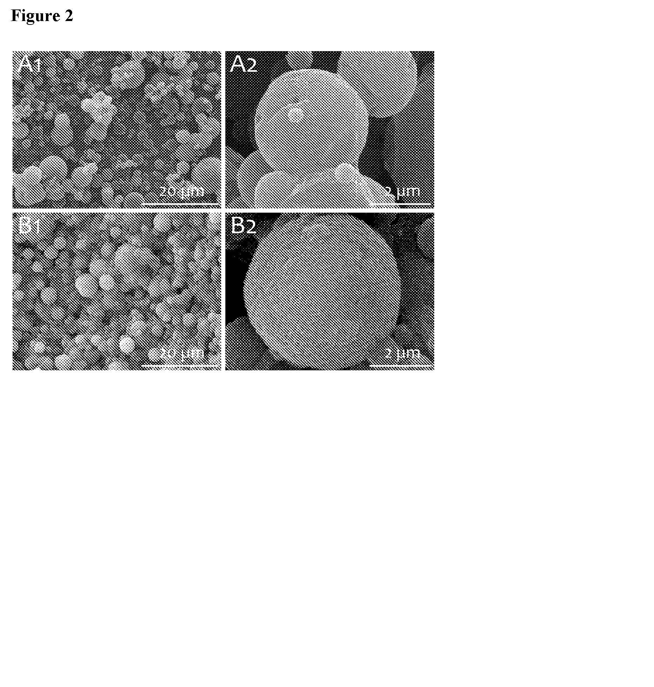

Figure 4. Confocal microscopy images of bone marrow dendritic cells incubated with OVA loaded mannitol/DS/OVA/P_{L}ARG microspheres.
Figure 5. X-ray diffractograms of pure mannitol, a physical mixture of mannitol/DS/OVA/P_{L}ARG and spray dried mannitollDS/OVA/P_{L}ARG microspheres.
Figure 6. *(A) Quantification of the relative enzymatic activity by conversion of 2,2'-azino-Bis(3-Ethylbenzothiazoline-6-Sulfonic acid) diammonium salt (ABTS) by free horseradish peroxidase (HRP) in solution, an aqueous dispersion of mannitol, polyelectrolyte and HRP and spray-dried microparticles after resuspension in phosphate buffered saline (PBS). (B) Confocal microscopy image showing the conversion of Amplex Red to resorufin (red fluorescence) within the spray-dried microspheres. after addition of H₂O₂.
Figure 7. Scanning electron microscopy images of spray-dried microparticles consisting of (A) HRP/DS/P_{L}ARG, thus without mannitol and (B) HRP/mannitol, this without the polyelectrolytes DS/P_{L}ARG.
Figure 8. X-ray powder diffractograms of HRP co-spray-dried with mannitol Without polyelectrolytes DS/P_{L}ARG, the mannitol clearly remained crystalline.

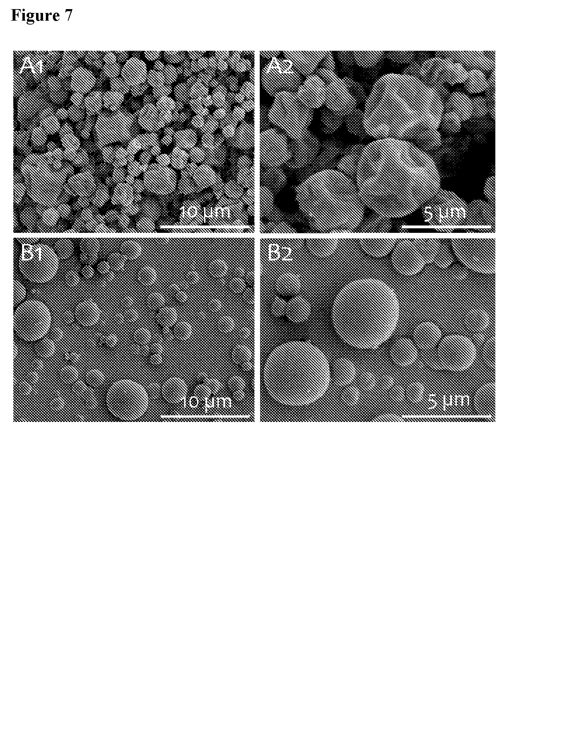

Figure 9. Assessment of cellular uptake of the spray-dried polyelectrolyte microspheres by dendritic cells. (A-B) Confocal microscopy images of dendritic cells incubated with spray-dried polyelectrolyte microspheres loaded with alexa488 conjugated ovalbumin (green fluorescence). In row A the cellular cytoplasm was stained with CellTracker red (red fluorescence). In row B the intracellular acidic vesicles (phagosomes, endosomes, lysosomes) were stained with LysoTracker Red (red fluorescene). In both cases the cell nuclei were stained with Hoechst (blue fluorescence). Column 1 shows the overlay between the blue, green and red channel, column 2 shows the overlay between the blue, green, red and DIC (differential interference contrast) channel and column 3 show the DIC channel. (C) Transmission electron microscopy images of spray-dried polyelectrolyte microspheres internalized by dendritic cells after (C1-C2) 4 hours and (C3-C4) 24 hours of incubation.
Figure 10. Cross-presentation of the SIINFEKL OVA-CD8 peptide measured by FACS analysis on 25-D1.16 mAb stained DCs that were incubated with soluble OVA, OVA encapsulated in PLGA microspheres, OVA encapsulated in hollow LbL capsules and OVA encapsulated in spray-dried polyelectrolyte microspheres. Experiments were run in triplicate. The polymers used for fabricating the LbL capsules were the same as those used for the spray-dried polyelectrolyte microspheres (i.e. dextran sulfate and poly-L-arginine).
Figure 11. Antibody titers and T cell induction (ELISPOT) of mice vaccinated with respectively soluble OVA (sOVA) and OVA formulated in spray dried microparticles (OVA particles).
Figure 12. Dual antigen release exhibited by different formulations of the invention upon redispersion of the dry powder (i.e. spray dried polyelectrolyte microspheres) where part of the antigen is retained within the polyelectrolyte framework (i.e. remains stably encapsulated within the microspheres) while another part is released into the surrounding medium. The ratio of mannitol/polyanion/polycation/antigen (using OVA as model antigen) is shown above each graph A-D.
Figure 13. (A) Size distribution of the spray dried microparticles upon reconstitution in water. (B) Scanning electron microscopy image of the spray dried microparticles.

### Description of the invention

The present invention discloses freeze-dried microspheres comprising a polyelectrolyte complex, an antigen and a polyol, characterised in that said polyelectrolyte complex is a complex of dextran sulphate and poly-L-arginine and said polyol is mannitol, and whrein said mannitol is in amorphous form.

The term "polyelectrolyte complex" refers to a neutral polymer-polymer complex composed of macromolecules carrying charges of opposite sign causing the macromolecules to be bound together by electrostatic interactions. The complex is usually formed by the electrostatic interaction of oppositely charged polyelectrolytes, such as an anionic polyelectrolyte or polyanion, and a cationic polyelectrolyte or polycation.

The polyelectrolyte complex preferably comprises i) a polyanion or an ampholyte with overall negative charge at a pH above the isoelectric point, and ii) a polycation or an ampholyte with overall positive charge at a pH below the isoelectric point.

Polyelectrolytes are polymers whose repeating units, or at least one of the repeating units, bear an electrolyte group. An electrolyte is any substance dissociating in an appropriate medium (e.g. water) into ions of opposite charge. A polymer is a macromolecule composed of repeating structural units connected by covalent chemical bonds. There are heteropolymers (comprising two or more kinds of structural units) and homopolymers (comprising only one kind of structural units). A polyelectrolyte may contain negatively and positively charge groups. These polyelectrolytes are called ampholytes. A skilled person knows that the net charge of an ampholytic compound in aqueous solutions depends on its isoelectrical point and on the pH of the solution. A polycation or cationic polyelectrolyte has an overall positive charge and comprises structural units with a net positive charge, whereas polyanions or anionic polyelectrolytes have an overall negative charge and comprises structural units with a net negative charge. A polyelectrolyte may also be considered as comprising a polyacid and a polybase.

In one embodiment disclosing any one of the microspheres of the present invention, the polyelectrolyte complex comprises an anionic polyelectrolyte and a cationic polyelectrolyte.

The polyanion or anionic polyelectrolyte is dextran sulfate. These polyelectrolytes are commercially available.

The polycation or cationic polyelectrolyte *is poly-L-arginine.* These polyelectrolyte are commercially available.

In one embodiment disclosing any one of the microspheres of the present invention, the polyelectrolyte complex is selected from the combinations of dextran sulfate and *poly-L-arginine.*

In one embodiment disclosing any one of the microspheres of the present invention, the ratio of polyanion to polycation in a polyelectrolyte complex may range from 2:1 (w/w) to 1:2 (w/w) (i.e. from two times the amount of polyanion relative to one amount of polycation to one amount of polyanion relative to two amounts of polycation), preferably from 1.5:1 1 to 1:1.5, more preferably from 1:1 to 1:0.5, even more preferably from 1:1 to 1:0.2.

The polyelectrolyte complex may additionally comprise inorganic or organic ions or salts, e.g. sodium chloride and antifoam agents, such as polypropylene glycol, polyethylene glycol, polyvinyl alcohol, and non-complexed polymers.

The term "polyol" refers to that compound with multiple hydroxyl groups, and includes sugars (reducing and non-reducing sugars), sugar alcohols, and sugar acids. A "reducing sugar" is one which contains a hemiacetal group that can reduce metal ions or react covalently with lysine and other amino groups in proteins. A "non-reducing sugar" is one which does not have these properties of a reducing sugar. Examples of suitable polyols for the present invention include mannitol. These polyols are commercially available.

Mannitol exists in three crystalline polymorphic forms: α, β and δ (see for example Burger, A., Henc, J. Rollinger, J., Weissnicht, A., Stottner, H. J. Pharm Sci, 89, 457, (2000)), and it is also found as a hemihydrate and in amorphous form. In the present invention, it is found in amorphous state after spray-drying a mixture of a polyelectrolyte complex and mannitol.

In one embodiment disclosing any one of the microspheres of the present invention, the amount of polyol in respect of the drug ranges from 20 to 40 times the amount of drug (w/w). Preferably within this embodiment, the polyol is mannitol.

In one embodiment, the microspheres above further comprises one or more drugs, wherein each drug has a molecular weight of at least 1000 Dalton.

As such, the polyol in combination with the polyelectrolyte complex and the one or more drugs are directly spray-dried, forming a polyelectrolyte framework that entraps the drug(s) and the polyol.

The drugs that can be formulated in the microspheres of the present invention, each with a molecular weight of at least 1000 Dalton, are typically biologic drugs. The term "biologic drugs" or "biologics" refers to complex pharmaceutical substances, preparations, or agents of organic origin, usually obtained by biological methods or assays.

In one embodiment disclosing any one of the microspheres of the present invention, the one or more drugs are biological factors such as antigens.

In one embodiment, the present invention discloses a composition comprising:
- an amorphous polyol,
- a polyelectrolyte complex, and
- one or more drugs, each with a molecular weight of at least 1000 Dalton;
wherein the amorphous polyol is amorphous mannitol, the polyelectrolyte complex is composed of dextran sulfate and *poly-L-arginine, and the one or more drugs is an antigen, for example ovalbumin.*

*In another* embodiment, in the composition of the preceding paragraph, the amorphous polyol has been extracted, thereby resulting in a porous composition, where the extraction is performed with water or any aqueous solution or dispersion. *In another* embodiment, the extraction of the amorphous polyol is performed in the body.

In one embodiment disclosing any one of the compositions of the present invention, the polyelectrolyte complex, the amorphous polyol, and optionally the one or more drugs, are homogeneously distributed.

The term "homogeneously distributed" means that the components occur at a substantially constant ratio to each other on any given area of the composition. The composition may contain areas of higher or lower concentration of the components, but the ratio of each to the other should not substantially vary. The term "components" refers to the polyelectrolyte complex, the amorphous polyol, and optionally the one or more drugs. Thus, in one embodiment, the polyelectrolyte complex, the amorphous polyol, and the one or more drugs, are homogeneously distributed. In another embodiment, the polyelectrolyte complex and the amorphous polyol, are homogeneously distributed

In the case of a composition of the present invention in particle form, this "any given area of the composition" refers both to the inner areas and to the outer surface of the particle, said particle being porous or non porous.

In one embodiment disclosing any one of the microspheres of the present invention, the amorphous polyol, the polyelectrolyte complex, and the one or more drug are present in a ratio (w/w/w, respectively) of ranges from around 1000/100/1 to around 1/100/1000, more preferably in a ratio from around 100/10/1 to around 1/10/100, even more preferably in a ratio from around 50/10/1 to around 1/10/50. In one embodiment relating to any one of the compositions of the present invention, the ratio is approximately 200:50:5 or 100:50: 5.

In one embodiment disclosing any one of the compositions of the present invention, said composition is in a particle form, or a powder.

In one embodiment disclosing any one of the compositions of the present invention, said composition is in a particle form, or a powder, and exhibits a dual drug release when provided in a suspension.

In one embodiment disclosing any one of the compositions of the present invention, said composition exhibits a dual drug release.

The term "powder" refers to two or more particles. These particles usually flow freely when shaken or tilted, but may also experience certain conglomeration forming a granular material within acceptable degrees.

In one embodiment disclosing any one of the compositions of the present invention, the particle has an average diameter between 100 nm and 100 µm.

In one embodiment disclosing any one of the compositions of the present invention, the particle has an average diameter between 100 nm and 10 µm.

In one embodiment disclosing any one of the compositions of the present invention, the particle has an average diameter between 1 µm and 10 µm, preferably between 1 µm and 5 µm.

The term "average diameter", in relation to the particle, which term can be used interchangeably with the phrases "average particle size" and "mean particle size", refers to the particle size of at least 50% or more, such as at least 60%, 70%, 80%, 90% of the particles of a given powder, when measured by standard techniques such as dynamic light scattering or laser diffraction. The term "average diameter" also refers to the larger dimension of a particle when the particle is not spherical in shape. In an exemplary embodiment, an average diameter between 100 nm and 100 µm means that at least 50% of the powder particles have a particle size from about 100 nm to about 100 µm when measured by standard techniques.

The present invention further discloses a suspension comprising the composition according to any one of the embodiments presented herein. Preferably, the suspension comprises any one of the composition of the present invention in particle form, or in powder.

In one embodiment of the invention, the suspension according has dual drug release properties.

The term "suspension" refers to dispersions of a solid internal face throughout an external phase that can be solid, liquid, or gas. Suspensions can be mono- or polydisperse. The dispersion occurs through mechanical agitation, optionally with the use of certain excipients or suspending agents, such as humectants, *flocculating agents, dispersant agents, gelling agents, and the like.* The solid internal phase is any one of the compositions of the present invention. When said composition is dispersed in a solid external phase, the obtained suspension is also termed as a powder, this last term being different from the "powder" defined above in relation to the particles of the present invention. When said composition is dispersed in a liquid external phase, the obtained suspension is termed as a suspension or as a colloid or colloidal system. When said composition is dispersed in a gas external phase, the obtained suspension is also termed as aerosol.

In one embodiment, the suspension comprises a composition in particle form or as a powder, where the amorphous polyol, acting as a sacrificial component, has been extracted. One example of such composition comprised in a suspension is that composition in particle form or as a powder where *the* extraction of the amorphous polyol is performed with a complexing agent such as water or any aqueous solution, thereby resulting in porous particles. One example is that composition in particle form or as a powder, comprised in a suspension, wherein the polyol is found in the external aqueous phase of the suspension.

As such, one embodiment of the present invention discloses a porous composition comprising a polyelectrolyte complex and one or more drugs. In one embodiment relating to the compositions of the preceding sentence, the polyelectrolyte complex, the one or more drugs, and the pores are homogeneously distributed. The pores are a result of the extraction of the amorphous polyol.

The term "sacrificial component", also known as "sacrificial template", and any other synonymous terms known by the skilled in the art, refer to a material or a portion thereof that can be extracted following the formation of the composition of the present invention. Interestingly, this subsequent extraction of the sacrificial component leads to porous microspheres having a network of connected pores, like those seen in spongy or coral structures. This particular porous structure allows easy access to the drug(s) and processing upon internalization by dendritic cells.

In the present invention, the sacrificial component is an amorphous polyol, or a polyol if found in solution, as defined herein above.

The extraction of the sacrificial component, i.e. the (amorphous) polyol, which can be the process of consumption, exhaustion, or removal may be performed using chemical, electrical, or other methods and combinations thereof, as conventionally used by the skilled in the art. Examples of chemical methods of extraction of sacrificial components include adding a complexing agent, reducing agent, oxidizing agent, hydrolyzing agent, and the like.

The term "complexing agent" refers to any material which binds or complexes selectively with the sacrificial component of the present invention, i.e. the amorphous polyol, or a polyol as such if in solution. This complexing agent may be a biological molecule such as a peptide or antibody, or a chemical agent, e.g. phosphotungstic acid (PTA), which may be in the form of a salt, e.g. sodium phosphotungstate. The complexing agents may be used singly or in combination. For example, a biological complexing agent may be used in tandem with a chemical complexing agent, such as the use of a peptide and a chemical agent. In another example, two complexing agents of the same class can be used together, e.g. a mixture of phosphotungstic acid (and salts thereof) and trichloroacetic acid. The complex formed must provide some means for separating the complex from the remainder of the composition, such as immobilization of the complexing agent to a surface. For example a complexing agent binds to the desired sacrificial component forming an agent/sacrificial component complex which has a higher specific gravity than the agent or the sacrificial component alone. Accordingly, the agent/ sacrificial component complex can be separated away via gravity which is optionally supplemented by the use of centrifugation.

In one embodiment disclosing any one of the compositions of the present invention, where the sacrificial component is mannitol or amorphous mannitol, the sacrificial component is extracted with a suitable complexing agent selected from water, a physiological solution, an aqueous solution or dispersion, and the like.

In one embodiment, the suspension comprises a composition in particle form or as a powder, where the amorphous polyol, acting as a sacrificial component, has not yet been extracted. One example of such composition comprised in a suspension is that composition in particle form or as a powder where the polyol is amorphous mannitol. The extraction of the amorphous mannitol is performed later on when the particles or powder are administered in the body, and the physiological water of the body itself acts as the extracting agent, thereby resulting in porous particles in situ, meaning inside the body. In one embodiment, the particles or powder comprising amorphous mannitol, polyelectrolyte complex, and one or more drugs, are suspended in a nonaqueous external phase, and this suspension is administered to the body where the physiological water shall extract the mannitol in situ.

In one embodiment, the suspension is formed in the body when the composition in particle form or as a powder is administered to the body, and the physiological aqueous solution of the body acts as external phase.

In another embodiment, the suspension starts forming in an extemporaneous preparation of the suspension, just before administration to the body, when the composition in particle form or as a powder is mixed with a physiological aqueous solution acting as external phase.

The present invention further discloses an aqueous feed comprising:
- mannitol,
- a polyelectrolyte complex of dextran sulphate and poly-L-arginine, and
- one or more antigens;
in a total solid concentration of about 0.1 to 10%.

Preferably, the total solid concentration ranges between 0.1 and 5%, more preferably between 0.5 and 2%, more preferably around 1%.

The term "aqueous feed" refers to any mixture with water.

The present invention further discloses a method for the preparation of the composition according to any one of the embodiments presented herein, said method comprising:
a) preparing an aqueous feed as defined above at a total solid concentration of about 0.1 to 10%;
b) spray-drying the aqueous feed prepared in step a).

The present invention further discloses a particle obtainable according to the method of the preceding paragraph.

The present invention further discloses a method for the preparation of the composition according to any one of the embodiments presented herein, said method comprising:
a) preparing an aqueous feed as defined above at a total solid concentration of about 0.1 to 10%;
b) spray-drying the aqueous feed prepared in step a); and
c) optionally extracting the polyol with a suitable complexing agent.

The present invention further discloses a particle obtainable according to the method of the preceding paragraph. This particle will be porous if the polyol has been conveniently extracted.

In the spray-drying technique, the aqueous feed of any one of the embodiments of the present invention is sprayed through the nozzle of a spray dryer whereby the aqueous solvent from the resulting droplets is evaporated, usually at elevated temperatures.

The spray-drying is conducted in a conventional spray-drying apparatus comprising a spray-drying chamber, atomizing means for introducing the feed mixture into the spray-drying chamber in the form of droplets, a source of heated drying gas that flows into the spray-drying chamber through an inlet, and an outlet for the heated drying gas.

The drying air may be any gas. Preferably, the gas is air, compressed air, or an inert gas such as nitrogen, nitrogen-enriched air, or argon. The temperature of the drying gas at the gas inlet of the spray-drying chamber can be from about 25 °C to about 300 °C or from about 60 °C to about 250 °C.

The spray-drying apparatus also comprises a means for collecting the solid pharmaceutical powder that is produced.

The atomizing means can be a rotary atomizer, a pneumatic nozzle, an ultrasonic nozzle or, preferably, a high-pressure nozzle.

Suitable rotary atomizers include those having an air turbine drive operating from a high pressure compressed air source, for example a 6 bar compressed air source, which supplies power to an atomization wheel for atomizing the feed mixture. The atomization wheel may be vaned. Preferably, the rotary atomizer is located in the upper part of the spray-drying chamber, for example in the chamber roof, so that the droplets produced dry and fall to the lower part of the chamber. Rotary atomizers produce droplets whose size depends upon the wheel peripheral velocity.

Suitable pneumatic nozzles (including two-fluid nozzles) comprise those that are located in the upper part of the spray-drying chamber, for example in the chamber roof, and operate in so-called "co-current mode". The feed mixture and the atomizing gas are passed separately to the nozzle head, where the atomization takes place. The size of the droplets produced by pneumatic nozzles depends on the operating parameters.

Two-fluid nozzles that operate in so-called "counter-current mode" may also be used. These nozzles operate in a similar way to two-fluid nozzles in co-current modes except that they are located in a lower part of the drying chamber and spray droplets upwards.

Suitable ultrasonic atomizer nozzles convert low viscosity liquids into ultra fine sprays. As liquids are pumped through the center of the probe, the liquids are mechanically pulverized into droplets from the vibrating tip. These droplets are larger with low frequency probes and smaller with higher frequency probes.

A preferred atomizer type for use in the invention is the high-pressure nozzle where liquid feed is pumped to the nozzle under pressure. Pressure energy is converted to kinetic energy, and feed issues from the nozzle orifice as a high-speed film that readily disintegrates into a spray as the film is unstable. The feed is made to rotate within the nozzle using a swirl insert or swirl chamber resulting in cone-shaped spray patterns emerging from the nozzle orifice. Swirl insert, swirl chamber and orifice dimensions together with variation of pressure gives control over feed rate and spray characteristics. The size of the droplets produced by high-pressure nozzles depends on the operating parameters.

Suitable atomizing means may be selected depending on the desired droplet size, which depends on a number of factors, such as the viscosity and temperature of the feed mixture, the desired flow rate and the maximum acceptable pressure to pump the feed mixture, have on droplet size. After selecting the atomizing means so that the desired average droplet size is obtained for a feed mixture having a particular viscosity, the mixture is admitted to the spray-drying chamber at a particular flow rate.

In one embodiment disclosing any one of the methods of the present invention, the spray-drying is performed in a spray dryer with a nozzle of a mean diameter range from around 0.1 to 10 mm, such as around 0.7 mm, 1.4 mm, or 2 mm.

Other spray-drying techniques known by the skilled in the art may be readily applied in the present invention. For example, nano spray-drying can be employed in which drying gas enters the system via the heater. A new kind of heater system allows for laminar air flow. The spray head sprays the fine droplets with a narrow size distribution into the drying chamber. The droplets dry and become solid particles. The solid particles are separated in the electrostatic particle collector. The exhaust gas is filtered and sent to a fume hood or the environment. The inlet temperature is controlled by a temperature sensor. Other spray-drying techniques include, for instance, monodisperse spray drying technology in which conventional spray drying is combined with microsieve™ nozzle technology to produce well-defined functional micro- and nanoparticles.

The present invention further relates to a pharmaceutical composition comprising the composition according to any one of the embodiments of the present invention and a pharmaceutical acceptable carrier, wherein the pharmaceutical composition comprises a pharmaceutically effective amount of one or more drugs.

In one embodiment, the pharmaceutical composition comprises a composition of the present invention in particle form or as a powder and a pharmaceutical acceptable carrier, wherein the pharmaceutical composition comprises a pharmaceutically effective amount of one or more drugs.

In one embodiment relating to any one of the pharmaceutical compositions of the present invention, said pharmaceutical composition is administered via the parenteral route selected from pulmonar, intravenous, intramuscular, subcutaneous, intraperitoneal, intra-articular, intralesional, intraventricular, by spinal injection, by intraosseous infusion, or transdermal routes. The pharmaceutical composition of the present invention may as well be administered intracavernously, intramyocardially, adventitially, intratumorally, at an intracerebral portion, at a wound site, at tight joint spaces, or at a body cavity of a human or animal.

The parenteral formulations may take such forms as suspensions in oily or aqueous vehicles, and may contain formulatory agents such as pH modifiers, complexing, isotonizing, suspending, stabilizing, or dispersing agents. In general, those excipients typically used for intravenous injections may be used in the formulations of the present invention as long as the formulation presents an appropriate viscosity, a reduced injection volume, and an acceptable pH range.

Methods of preparing various pharmaceutical compositions with a certain amount of active ingredient are known to those skilled in this art. For examples of methods of preparing pharmaceutical compositions, see Remington; The Science and Practice of Pharmacy, Lippincott Williams & Wilkins, 21st Ed, 2006.

Preferably, the pharmaceutical composition is presented in powder form for later constitution with a suitable vehicle, e.g. sterile pyrogen-free water before use. The extemporaneous unit dosage forms are prepared by admixing a composition of the invention, preferably in particle form or as powder or as suspension thereof, and a sterile vehicle. Alternatively, the composition can be suspended for injection and sterilized before filling into a suitable vial or ampoule and sealing. Advantageously, excipients such as local anaesthetic preservatives and buffering agents are dissolved in the vehicle. A surfactant or wetting agent may be included in the composition to facilitate a uniform distribution of the composition.

Formulations for injection may be presented in unit dosage form e.g. in ampoules or in multidose containers. In one embodiment, one ampoule comprises the composition of the present invention in particle form or as a powder, and the other ampoule comprises the vehicle. In another embodiment, one ampoule comprises a suspension of the composition of the present invention in particle form or as a powder, ready for pulmonary delivery or ready for injection.

For purposes of transdermal (e.g., topical) administration, dilute sterile, aqueous or partially aqueous solutions (usually in about 0.1% to 5% concentration), otherwise similar to the above parenteral solutions, are prepared.

Therapeutically effective doses of the compounds within the compositions of the present invention required to prevent or to treat the medical condition are readily ascertained by one of ordinary skill in the art using preclinical and clinical approaches familiar to the medicinal arts. The dose of the compound or a pharmaceutically acceptable salts thereof to be administered depends on the individual case and, as customary, is to be adapted to the conditions of the individual case for an optimum effect. Thus it depends, of course, on the frequency of administration and on the potency and duration of action of the compound employed in each case for therapy or prophylaxis, but also on the nature and severity of the disease and symptoms, and on the sex, age, weight co-medication and individual responsiveness of the subject to be treated and on whether the therapy is acute or prophylactic. The percentage of drug present in the formulation is also a factor. Doses may be adapted in function of weight and for pediatric applications.

The present invention further relates to any one of the pharmaceutical compositions of the present invention, for use in extracellular drug delivery.

The present invention further relates to any one of the pharmaceutical compositions of the present invention, for use in the delivery of one or more drugs to phagocytes.

The term "phagocyte" refers to dendritic cells including Langerhans cells; macrophages such as epithelioid cells, foam cells, foreign-body giant cells, Langhans giant cells, histiocytes, Kupffer cells, alveolar macrophages, peritoneal macrophages; monocytes including activated killer monocytes; and neutrophils.

The present invention further relates to any one of the pharmaceutical compositions of the present invention, for use in the treatment of allergy- and immune-related diseases, dermatologic diseases, cardiac diseases, endocrine diseases, metabolic diseases, hematologic diseases, gastrointestinal diseases, colorectal diseases, infectious diseases, cancer, kidney diseases, pulmonary related diseases, rheumatologic diseases, neurological diseases, osteopathic diseases, orthopedic diseases, ophthalmological diseases, otolaryngology related diseases, urological diseases.

In one embodiment, any one of the pharmaceutical compositions of the present invention are for use in the treatment of immune-related diseases, infectious diseases, metabolic diseases, and cancer.

The following examples are intended to illustrate the present invention and not to limit it thereto.

### Examples

### Example 1: Synthesis of solid microspheres

Dextran sulphate (DEXS; 10 kDa), poly-L-arginine (P_{L}ARG, 100 kDa), ovalbumin (OVA; grade VII) and mannitol were obtained from Sigma-Aldrich. OVA-alexa488, and LysoTracker Red were obtained from Invitrogen. All water used in the experiments was of Milli-Q grade.

Mannitol was mixed with dextran sulfate (DS) and ovalbumin (OVA; used as model antigen in this work) under stirring, in water in a 40/4/1/5 ratio at a total solid concentration of 1 %. In detail, 200 mg mannitol, 20 mg DS and 5 mg OVA were dissolved in 20 ml water. Subsequently P_{L}ARG was dissolved in 5 ml water and added dropwise to the stirring mannitol/DS/OVA solution, to allow electrostatic complexation between P_{L}ARG and respectively DS and OVA.

Subsequently, the mixture was spray-dried using a Buchi B290 bench top spray-drier and collected as a dry powder. The mixture was fed to a two-fluid nozzle (diameter 0.7 mm) at the top of the spray dryer. In addition, the spray dryer operated in co-current air flow at 35 m³/h with an inlet drying air temperature of 130 °C and an outlet drying air temperature of 65 °C. These particles will be termed solid microspheres further on in this example.

### Example 2: Particle characterization

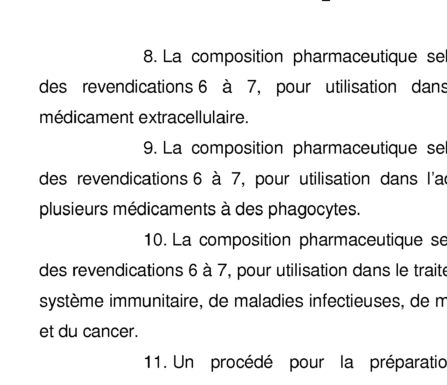

The morphology of (Figure 2A) the solid microspheres, and (Figure 2B) the porous microspheres obtained after extraction of the mannitol in water, was visualized by scanning electron microscopy (SEM).

Most importantly, as it will be addressed below in more detail by confocal microscopy, the spray-dried microspheres remained stable upon resuspension in aqueous medium and did not disassemble into the initial starting components. As a control we also spray-dried a mixture of mannitol and OVA and immediate dissolution of the micropartciles could be observed upon addition of water (data not shown), indicating the requirement of the DS/P_{L}ARG polyelectrolyte complex framework to obtain stable microspheres. Extraction of the mannitol in water led to the formation of porous microspheres which were most likely stabilized through a combination of electrostatic and hydrophobic interactions together with physical entanglements of the polymer and protein chains.

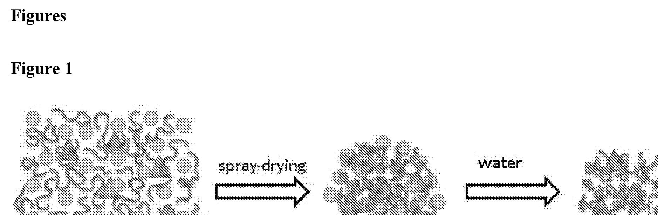

Confocal microscopy was used to visualize the microspheres in a hydrated state. Confocal microscopy images were recorded on a Leica SP5 AOBS confocal microscope. For visualization purpose, green fluorescent OVA-alexa488 was mixed with native OVA in a 50:1 ratio. The confocal micrographs in Figure 3 confirmed that the microspheres remained stable in aqueous medium. Importantly, when considering the green fluorescence channel of Figure 3A it was observed that the green fluorescent OVA-alexa488 was retained within the polyelectrolyte framework of the porous microspheres rather than being spontaneous released into the surrounding aqueous medium.

### Example 3: Determination of encapsulation efficiency

Quantification of the encapsulation efficiency was determined by re-suspending dry solid microspheres in respectively phosphate buffered saline (PBS; pH 7.4). Subsequently, the microspheres were centrifuged and the OVA concentration in the supernatant was determined. When defining the encapsulation efficiency as the amount of protein that is retained within the microspheres upon re-suspension, relative to the amount of protein in the dry microspheres, encapsulation efficiency of 100 ± 1 % upon re-suspension in PBS was observed. This number meant that upon re-suspension in PBS no detectable amounts of OVA were released from the porous microsphere upon extraction of the mannitol from the microspheres. An encapsulation efficiency of 100 % into the porous polyelectrolyte spheres was considerably higher than the approximately 50 %, reported earlier for encapsulation of OVA into hollow polyelectrolyte multilayer capsules [17]. In that case, OVA was co-precipitated into calcium carbonate microparticles by mixing it with calcium chloride and sodium carbonate. After LbL alternate coating of these microparticles with dextran sulfate and poly-L-arginine and subsequent dissolution of the CaCO₃ microparticles approximately 50 % of the OVA was lost due to diffusion through the polyelectrolyte membrane.

### Example 4: Dendritic cell uptake assessment

As it was the aim to use the porous polyelectrolyte microspheres as antigen carriers for vaccination purpose, it was important to assess whether these porous microspheres were able deliver their payload to dendritic cells.

Female C57BL/6 mice were purchased from Janvier and housed in a specified pathogen-free facility in micro-isolator units. Dendritic cells were generated using a modified Inaba protocol. Two to six months C57BL/6 mice were sacrificed and bone marrow was flushed from their femurs and tibias. After lysis of red blood cells with ACK lysis buffer (BioWhittaker), granulocytes and B cells were depleted using Gr-1 (Pharmingen) and B220 (Pharmingen) antibodies respectively, and low-toxicity rabbit complement (Cedarlane Laboratories Ltd.). Cells were seeded at a density of 2 x 10⁵ cells/ml in 175 cm² Falcon tubes (Becton Dickinson) in dendritic cell medium (RPMI 1640 medium containing 5% LPS-free FCS, 1% penicillin/streptomycin, 1% L-glutamine and 50 µM β-mercaptoethanol) containing 10 ng/ml IL-4 and 10 ng/ml GM-CSF (both from Peprotech). After two days and again after four days of culture, the non-adherent cells were centrifuged, resuspended in fresh medium and replated to the same falcons. On the sixth day, non-adherent cells were removed and fresh medium containing 10 ng/ml GM-CSF and 5 ng/ml IL-4 was added. On day 8 of culture, nonadherent cells were harvested and used for the experiments.

Differentiated murine dendritic cells were incubated with OVA-alexa488-loaded porous microspheres and followed by confocal microscopy imaging. In order to discriminate whether the microspheres were internalized by dendritic cells, the cell nuclei blue fluorescent were counterstained with HOECHST and the intracellular acidic vesicles were stained with LysoTracker. As both green fluorescent microspheres, cell nuclei and acidic vesciles were visible in the same confocal plane and the microspheres were clearly situated within the boundaries of the cell membrane, it could unambiguously be concluded that the microspheres were effectively internalized by the dendritic cells and located in intracellular acidic vesicles.

### Example 5: Assessing crystallinity of spray dried mannitol

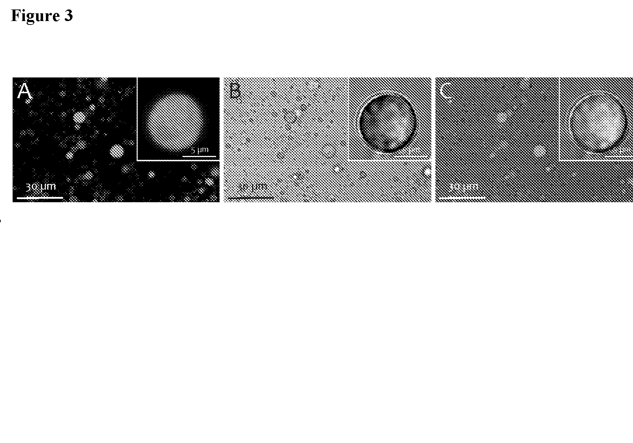

X-ray diffraction was used to assess the crystallinity of respective mannitol, a physical mixture of mannitol/OVA/DS/P_{L}ARG, and spray dried mannitol/OVA/DS/P_{L}ARG microspheres. Figure 5 clearly illustrates and demonstates that after spray-drying the mannitol is obtained in an amorphous form. This is important to enhance protein stability within the spray dried microparticles.

In conclusion, a facile method to encapsulate antigen into porous degradable polyelectrolyte microspheres is hereby demonstrated. The major advantage of this approach is the possibility to produce microspheres on a large scale involving a minimum of process steps combined with a high encapsulation efficiency that minimizes the waste of expensive antigen and polyelectrolytes. Moreover, the mannitol is retained in an amorphous form which is of particular importance regarding protein stability.

### Example 6: Preparation of microparticles with ovalbumin

An aqueous dispersion (1% w/w) was prepared by mixing mannitol, dextran sulfate (DS) and protein (e.g. ovalbumin (OVA) as model antigen) under stirring followed by dropwise addition of poly-L-arginine (P_{L}ARG) to allow electrostatic complexation between DS, OVA and P_{L}ARG. The ratio (w/w) of mannitol to OVA, DS and P_{L}ARG was chosen 40:1:4:5. Subsequently, the mixture was spray-dried using a lab-scale Buchi B290 spray-drier and collected as a dry. Upon addition of water the mannitol readily dissolves and stable microparticles remain consisting of a polyelectrolyte framework encapsulating the co-spray-dried protein.

### Example 7: Spray dried polyelectrolyte microspheres retain the biological activity of encapsulated proteins

For applications in drug delivery, the preservation of the biological activity of the encapsulated protein within the polyelectrolyte framework is of paramount importance. Therefore we encapsulated an enzyme, horseradish peroxidase (HRP), instead of an antigen and compared the enzymatic catalytic activity (*i.e.* rate of substrate conversion) by the free HRP enzyme in solution to the rates of conversion yielded by the aqueous mannitol/DS/HRP/P_{L}ARG dispersion and by the HRP loaded spray-dried microparticles. This was performed by following the increase in UV-VIS absorbance at 403 nm due to the conversion of the substrate ABTS (2,2'-azino-bis(3-Ethylbenzothiazoline-6-Sulfonic acid) diammonium salt) by HRP:

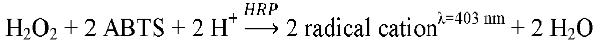

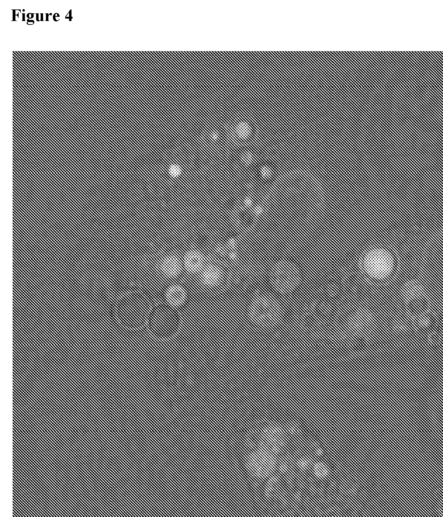

From the slope of the obtained kinetic curves, the enzymatic catalytic activity (Figure 6A) was derived. When the rate of conversion of ABTS by free HRP free solution was equal to 100%, an activity of 87 ± 3% for the physical mixture and 84 ± 5% for the spray-dried microparticles was calculated. This showed that upon mixing, a 13 ± 3% drop in enzymatic activity occurred which was likely due to electrostatic interaction with the DS/P_{L}ARG polyelectrolyte complexes. As HRP had an isoelectric point of ∼8.8, it was positively charged at neutral pH and was likely to undergo complexation with DS. Most strikingly, the spray drying process itself only induced a further activity decrease of merely 3 %.

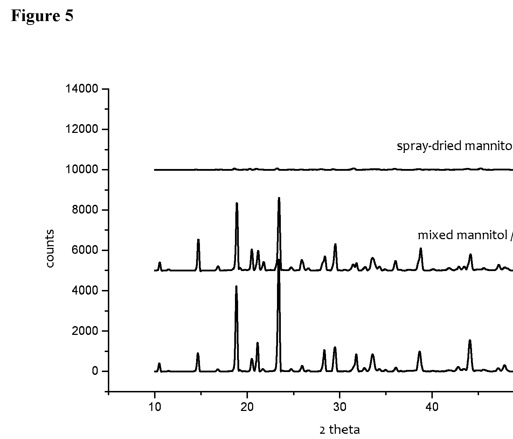

As a control we also measured the enzymatic activity of HRP spray-dried with DS/P_{L}ARG but without mannitol and the enzymatic activity of HRP spray-dried with mannitol but without the polyelectrolytes DS/P_{L}ARG. Without mannitol, bumped dense microparticles were obtained (see Supporting Information for SEM images (Figure 7) which could only be recovered at very low yields, most likely due to electrostatic interaction with the glass wall of the spray drying cylinder. The enzymatic activity of the encapsulated HRP was measured to be 50 ± 8 % which was significantly lower than in case of mannitol/DS/HRP/P_{L}ARG microparticles.

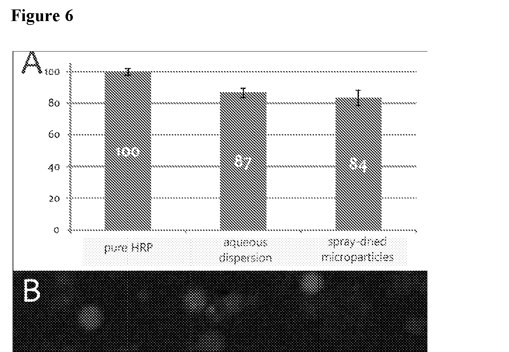

Microparticles consisting of solely HRP and mannitol, thus without polyelectrolytes, were also obtained as a perfectly spherically shaped powder (see supporting information for SEM images). They dissolved readily upon addition of water, yielding a solution rather than a microparticle suspension obtained in case of spray-dried mannitol/DS/HRP/P_{L}ARG microparticles. Moreover, the enzymatic activity of the encapsulated HRP was measured to be 50 ± 12 % which was again significantly lower than in case of mannitol/DS/HRP/P_{L}ARG microparticles. Taking into account that XRPD measurements on the mannitol/HRP microparticles (without polyelectrolytes) showed the mannitol to be in a crystalline state (see Supporting Information Figure 8) while the presence of polyelectrolytes yielded amorphous mannitol, we hypothized that the presence of amorphous mannitol strongly augmented the preservation of the enzymatic activity of encapsulated HRP

Thus the encapsulation approach demonstrated in this work allowed a much higher preservation (*i.e*. 84 ± 5%) of HRP activity compared to common LbL polyelectrolyte capsules or amphiphilic vesicles where typically a reduction of 50 to 85% of enzymatic activity upon encapsulation was observed. Moreover, these other encapsulation strategies suffered from far lower encapsulation efficiencies compared to our spray drying approach. To demonstrate that enzymatic reaction occurred inside the spray-dried microparticles we used Amplex Red as fluorogenic substrate instead of ABTS. In the presence of H₂O₂ Amplex Red is converted by HRP to resorufin which is strongly fluorescent. Figure 6B shows a confocal microscopy image after addition of Amplex Red and H₂O₂ to a mannitol/DS/HRP/P_{L}ARG microparticle suspension. The fluorescent microspheres indicate that reaction takes place inside the microspheres followed by diffusion of the dye, coloring the medium.

### Example 8: Spray dried polyelectrolyte microspheres are rapidly internalized by dendritic cells and their antigen payload is efficiently cross-presented via MHC class I

In a next series of experiments we investigated the interaction of the polyelectrolyte microspheres with dendritic cells (DCs) which are the primary target cell population for vaccine delivery. In this work we used DCs derived from bone marrow of mice. Figure 9A-B shows confocal microscopy images of DCs incubated with spray-dried microparticles loaded with OVA-alexa488 (green fluorescence). In Figures 9A the cellular cytoplasm was stained red fluorescent with CellTracker Red and in Figure 9B the intracellular acidic vesicles (phagosomes, endosomes, lysosomes) were stained red fluorescent with lysoTracker Red. From the overlay between the fluorescence image and the DIC (differential interference contrast) channel in Figure 9A2 it is evident that the polyelectrolyte microparticles became internalized. In Figures 9B1 co-localisation - expressed as a yellow/orange signal - between the green fluorescence of the polyelectrolyte microparticles and the red fluorescence of the intracellular vesicles is observed. For clarity of presentation we have marked in Figure 9B1 an internalized polyelectrolyte microparticle with an orange arrow and marked a non-internalized polyelectrolyte microparticle with a green arrow. These data indicate that upon internalisation the polyelectrolyte microparticles end up in intracellular acidic vesicles, which is common for most types of microparticles so far. More detail on the intracellular behaviour of the internalised microparticles was obtained by imaging ultrathin sections of epoxy embedded DCs by transmission electron microscopy (TEM). Figure 9C shows TEM images of DCs incubated for 4h (Figures 9C1 -9C2) and (Figures 9C3-9C4) 24h.

Following uptake, the particles were surrounded by a membrane (red arrow, Figure 9C2), which indicates that the particles ended up in phagolysosomal compartments following uptake, as was also previously described for hollow LbL capsules. These data are also in accordance with the observed co-localisation with Lysotracker Red, as shown in Figure 9B1. The TEM images in Figures 9C3-3C4 clearly demonstrate that after one day incubation the polyelectrolyte microparticles become deformed and the zoomed image in Figure 9C4 shows the recruitment of different intracellular organelles such as ER (blue arrow), mitochondria (green arrow) and lysosomes (yellow arrow) towards the deformed polyelectrolyte microparticle. These TEM data indicate an active intracellular processing of the internalized polyelectrolyte microparticles and puts them en route towards potential application in vaccine delivery.

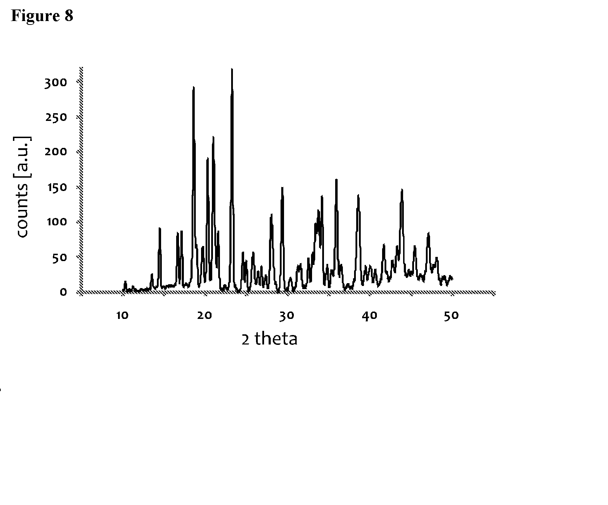

Next we aimed to assess whether the encapsulated OVA was still available for processing upon internalization by DCs and if so, whether the peptide fragments become cross-presented *via* an peptide-MHC class I complex. Following endocytosis, soluble exogenous antigens are generally cleaved by lysosomal proteases and presented *via* a peptide-MHC class II complex to CD4 T cells. DCs also harbor the capacity to present exogenous antigens *via* MHCI, a feature called cross-presentation that is necessary to prime CD8 cytotoxic T cells capable of killing virally infected cells. However, cross-presentation of soluble antigens occurs extremely inefficient, but is dramatically augmented when the antigen is in a particulate form, as was found for several types of microparticulate antigen carriers. To address whether this important feature also holds true for the spray-dried polyelectrolyte microspheres, we incubated DCs with equivalent amounts of soluble OVA and OVA encapsulated in either hollow LbL capsules, PLGA microspheres or spray-dried polyelectrolyte microspheres. After a 48h incubation period, DCs were stained with the 25-D1.16 mAb which specifically recognizes the SIINFEKL OVA CD8+ epitope complexed to MHC class I H-2Kb molecules. As shown in Figure 10, a dose-dependent increase in cross-presentation of encapsulated OVA was observed when compared to the soluble antigen. Spray-dried particles were significantly more potent than PLGA and hollow capsules LbL in stimulating antigen cross-presentation. This is most likely due to the fact that the spray-dried polyelectrolyte microspheres carry more antigen per particle compare to hollow LbL capsules and PLGA microspheres, thus delivering more antigen when a DC internalizes a particle. Moreover, the spray-dried polyelectrolyte microspheres allow readily processing of the encapsulated antigen due to their fully hydrated structure, which is not the case for PLGA microspheres which gradually release their payload through surface erosion based degradation and therefore are incapable of inducing antigen cross-presentation within the experimental timeframe of 48 h.

### Example 9: In vivo immunisations

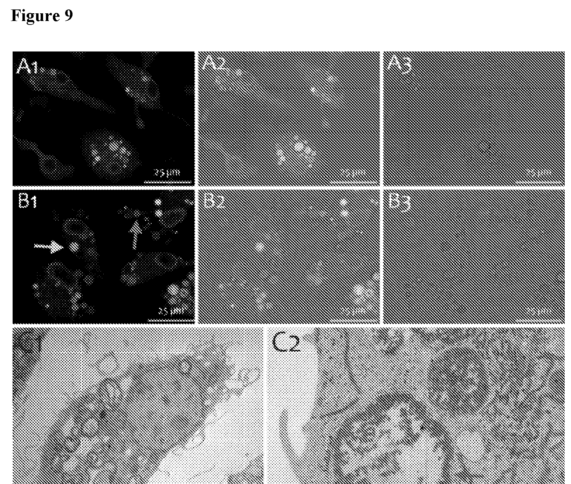

Mice were vaccinated with respectively soluble OVA (sOVA) and OVA formulated in spray dried polyelectrolyte microspheres (OVA particles). A prime boost was applied: with a time interval of 3 weeks, mice were injected subcutaneously with 50 µg OVA. Two weeks after each immunization, blood samples were withdrawn and antibody titers against IgG1 and IgG2c were measured by ELISA and shown in Figure 11. ELISPOT was performed on the spleens, 3 weeks after the last immunization, and the induction of OVA-specific CD4 and CD8 T-cells was measured and shown in Figure 11.

### Example 10: Dual release properties of spray dried polyelectrolyte microspheres

In this series of experiments we demonstrate that by varying the particle composition it is possible to obtain a dual antigen release. This dual antigen release is defined as follows. Upon redispersion of the dry powder (i.e. spray dried polyelectrolyte microspheres) part of the antigen is retained within the polyelectrolyte framework (i.e. remains stably encapsulated within the microspheres) while another part is released into the surrounding medium. This feature is of importance for the induction of humoral (antibody mediated) immune responses as for this purpose intact antigen should reach B-cells, either via passive drainage through the lymphatics or transported via DCs. As antigen, encapsulated within polyelectrolyte microspheres is likely to be processed (i.e. cleaved into peptide fragments) once internalized by DCs, it is unlikely that DCs will pass intact antigen (that was internalized under the form of polyelectrolyte microspheres) to B-cells. Therefore, a formulation that contains both encapsulated and soluble antigen should be capable to target, besides DCs with encapsulated antigen, also B cells with intact soluble antigen.

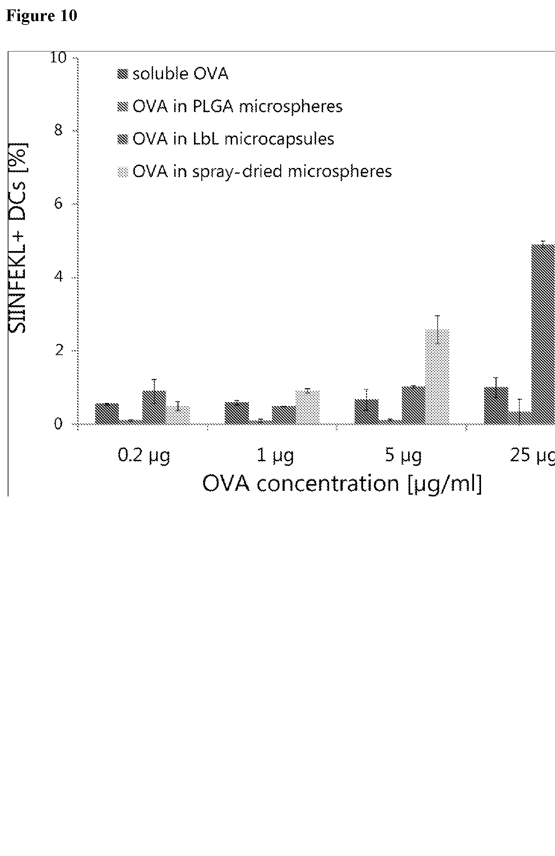

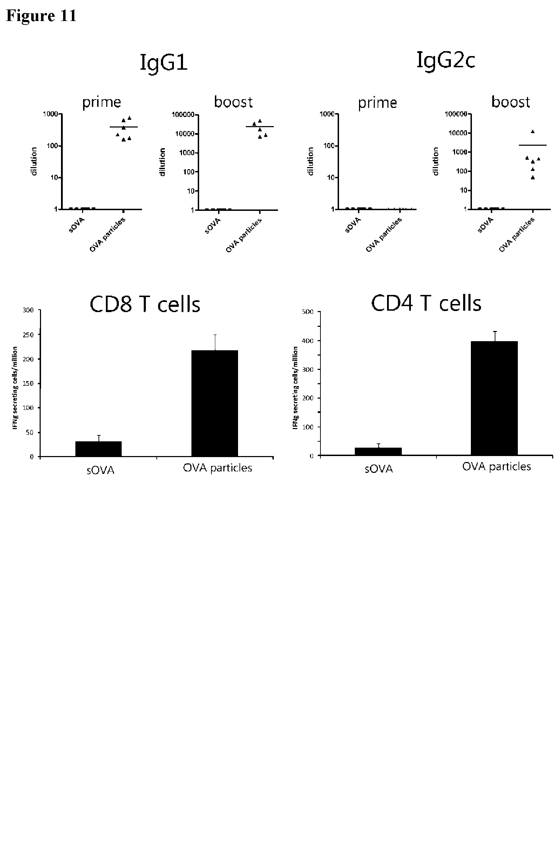

We found that the following parameters were crucial to tailor the dual release: type of polyelectrolyte, ratio polyanion to polycation, and amount of mannitol. This is illustrated by the Figures 12A-D. The ratio of mannitol/polyanion/polycation/antigen (using OVA as model antigen) is shown above each graph. The polyelectrolytes used for these experiments were DS: dextran sulfate - negatively charged polyelectrolyte; PIARG: poly-L-arginine - positively charged polyelectrolyte; Protamine - positively charged polyelectrolyte; and HEP: heparin - negatively charged polyelectrolyte.

### Example 11: Size reduction of spray dried microspheres below 5 µm

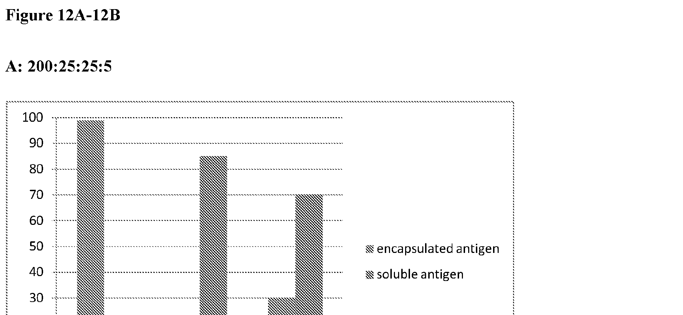

Microparticles with a size below 5 µm show enhanced uptake by dendritic cells in vivo. Therefore process parameters were optimized in order to reduce the size of the spray dried microspheres. As shown in Figure 13, this allowed us to obtain particles with a mean diameter of 3,5 µm and all of them being below 10 µm.

The process parameters that were optimized were as follows:
- Spray drying apparatus: Buchi B-290
- Inlet temperature: 130°C
- Outlet temperature: 58°C
- Feed flow: 1.3 ml/min
- Feed concentration: 1 %.

### References

1. Kovacsovicsbankowski, M.; Clark, K.; Benacerraf, B.; Rock, K. L. Proc. Natl. Acad Sci. U.S.A. 1993, 90, 4942-4946.
2. Guermonprez, P.; Saveanu, L.; Kleijmeer, M.; Davoust, J.; van Endert, P.; Amigorena, S. Nature 2003, 425, 397-402.
3. Shen, Z. H.; Reznikoff, G.; Dranoff, G.; Rock, K. L. J. Immunol. 1997, 158, 2723-2730.
4. Sousa, C. R. E.; Germain, R. N. J Exp. Med. 1995, 182, 841-851.
5. F. Caruso, R. A. Caruso, H. Mohwald, Science 1998, 282, 1111.
6. E. Donath, G. B. Sukhorukov, F. Caruso, S. A. Davis, H. Mohwald, Angewandte Chemie-International Edition 1998, 37, 2202.
7. G. B. Sukhorukov, E. Donath, S. Davis, H. Lichtenfeld, F. Caruso, V. I. Popov, H. Mohwald, Polymers for Advanced Technologies 1998, 9, 759.
8. G. B. Sukhorukov, H. Mohwald, Trends in Biotechnology 2007, 25, 93.
9. C. S. Peyratout, L. Dahne, Angewandte Chemie-International Edition 2004, 43, 3762.
10. G. Skirtach, A. M. Javier, O. Kreft, K. Kohler, A. P. Alberola, H. Mohwald, W. J. Parak, G. B. Sukhorukov, Angewandte Chemie-International Edition 2006, 45, 4612.
11. G. Decher, Science 1997, 277, 1232.
12. T. Boudou, Y. Crouzier, K. Ren, G. Blin, C. Picart, Advanced Materials 2010, 22, 441.
13. B. G. De Geest, S. De Koker, G. B. Sukhorukov, O. Kreft, W. J. Parak, A. G. Skirtach, J. Demeester, S. C. De Smedt, W. E. Hennink, Soft Matter 2009, 5, 282.
14. B. G. De Geest, N. N. Sanders, G. B. Sukhorukov, J. Demeester, S. C. De Smedt, Chemical Society Reviews 2007, 36, 636.
15. B. G. De Geest, G. B. Sukhorukov, H. Mohwald, Expert Opinion on Drug Delivery 2009, 6, 613.
16. J. F. Quinn, A. P. R. Johnston, G. K. Such, A. N. Zelikin, F. Caruso, Chemical Society Reviews 2007, 36, 707.
17. S. De Koker, B. G. De Geest, S. K. Singh, R. De Rycke, T. Naessens, Y. Van Kooyk, J. Demeester, S. C. De Smedt, J. Grooten, Angewandte Chemie-International Edition 2009, 48, 8485.
18. R. De Rose, A. N. Zelikin, A. P. R. Johnston, A. Sexton, S. F. Chong, C. Cortez, W. Mulholland, F. Caruso, S. J. Kent, Advanced Materials 2008, 20, 4698.
19. R. Palankar, A. G. Skirtach, O. Kreft, M. Bedard, M. Garstka, K. Gould, H. Mohwald, G. B. Sukhorukov, M. Winterhalter, S. Springer, Small 2009, 5, 2168.
20. Sexton, P. G. Whitney, S. F. Chong, A. N. Zelikin, A. P. R. Johnston, R. De Rose, A. G. Brooks, F. Caruso, S. J. Kent, Acs Nano 2009, 3, 3391.
21. S. F. Chong, A. Sexton, R. De Rose, S. J. Kent, A. N. Zelikin, F. Caruso, Biomaterials 2009, 30, 5178.
22. S. De Koker, T. Naessens, B. G. De Geest, P. Bogaert, J. Demeester, S. De Smedt, J. Grooten, Journal of Immunology 2009, 50 ± 184, 203.
23. O. E. Selina, S. Y. Belov, N. N. Vlasova, V. I. Balysheva, A. I. Churin, A. Bartkoviak, G. B. Sukhorukov, E. A. Markvicheva, Russian Journal of Bioorganic Chemistry 2009, 35, 103.
24. D. V. Volodkin, N. I. Larionova, G. B. Sukhorukov, Biomacromolecules 2004, 5, 1962.

## Claims

1. Freeze-dried microspheres comprising a polyelectrolyte complex, an antigen and a polyol, **characterised in that** said polyelectrolyte complex is a complex of dextran sulphate and poly-L-arginine, and said polyol is mannitol, and wherein mannitol is in amorphous form.

2. The freeze-dried microspheres according to claim 1, wherein the polyelectrolyte complex, the amorphous mannitol, and the antigen, are homogeneously distributed.

3. The freeze-dried microspheres according to claims 1 or 2, wherein the amorphous mannitol, the polyelectrolyte complex, and the antigen are present in a ratio (w/w/w, respectively) of ranges from around 1000/100/1 to around 1/100/1000.

4. A suspension comprising the freeze-dried microspheres according to any one of claims 1-3.

5. The suspension according to claim 4, wherein said suspension has dual drug release properties.

6. A pharmaceutical composition comprising the freeze-dried microspheres according to any one of claims 1-3 and a pharmaceutical acceptable carrier, wherein the pharmaceutical composition comprises a pharmaceutically effective amount of the antigen.

7. The pharmaceutical composition according to claim 6, wherein said pharmaceutical composition is administered via the parenteral route.

8. The pharmaceutical composition according to any one of claims 6-7, for use in extracellular drug delivery.

9. The pharmaceutical composition according to any one of claims 6-7, for use in the delivery of one or more drugs to phagocytes.

10. The pharmaceutical composition according to any one of claims 6-7, for use in the treatment of immune-related diseases, infectious diseases, metabolic diseases, and cancer.

11. A method for the preparation of freeze-dried microspheres according to any one of claims 1-3, said method comprising:
a) preparing an aqueous feed of the polyelectrolyte complex, the antigen and the polyol as defined in claim 1 at a total solid concentration of about 0.1 to 10%,
b) spray-drying the aqueous feed prepared in step a).

## Patentansprüche

1. Gefriergetrocknete Mikrokügelchen, welche einen Polyelektrolytkomplex, ein Antigen und ein Polyol umfassen, **dadurch gekennzeichnet, dass** der erwähnte Polyelektrolytkomplex ein Komplex von Dextransulfat und Poly-L-Arginin ist, und dass das erwähnte Polyol Mannitol ist, und wobei Mannitol in amorpher Form vorliegt.

2. Die gefriergetrockneten Mikrokügelchen nach Anspruch 1, wobei der Polyelektrolytkomplex, das amorphe Mannitol, und das Antigen, homogen verteilt sind.

3. Die gefriergetrockneten Mikrokügelchen nach Ansprüchen 1 oder 2, wobei das amorphe Mannitol, der Polyelektrolytkomplex, und das Antigen in einem Verhältnis (Gew./Gew./Gew., respektive) von Bereichen von etwa 1.000/100/1 bis etwa 1/100/1.000 vorhanden sind.

4. Eine Suspension, welche die gefriergetrockneten Mikrokügelchen nach irgendeinem der Ansprüche 1 bis 3 enthält.

5. Die Suspension nach Anspruch 4, wobei die erwähnte Suspension duale Arzneimittelfreisetzungseigenschaften hat.

6. Eine pharmazeutische Zusammensetzung, welche die gefriergetrockneten Mikrokügelchen nach irgendeinem der Ansprüche 1 bis 3 und einen pharmazeutisch akzeptablen Träger enthält, wobei die pharmazeutische Zusammensetzung eine pharmazeutisch wirksame Menge des Antigens enthält.

7. Die pharmazeutische Zusammensetzung nach Anspruch 6, wobei die erwähnte pharmazeutische Zusammensetzung auf parenteralem Weg verabreicht wird.

8. Die pharmazeutische Zusammensetzung nach irgendeinem der Ansprüche 6 bis 7, zur Anwendung in extrazellulärer Arzneimittelabgabe.

9. Die pharmazeutische Zusammensetzung nach irgendeinem der Ansprüche 6 bis 7, zur Abgabe eines oder mehrerer Arzneimittel an Phagozyten.

10. Die pharmazeutische Zusammensetzung nach irgendeinem der Ansprüche 6 bis 7, zur Anwendung in der Behandlung von mit dem Immunsystem zusammenhängenden Erkrankungen, Infektionskrankheiten, Stoffwechselkrankheiten, und Krebs.

11. Ein Verfahren zur Zubereitung von gefriergetrockneten Mikrokügelchen nach irgendeinem der Ansprüche 1 bis 3, wobei das erwähnte Verfahren Folgendes umfasst:
a) Zubereitung einer wässrigen Speiselösung aus dem Polyelektrolytkomplex, dem Antigen und dem Polyol, wie definiert in Anspruch 1, in einer Feststoffkonzentration von etwa 0,1 bis 10%,
b) Sprühtrocknen der in Schritt a) zubereiteten wässrigen Speiselösung.

## Revendications

1. Des microsphères lyophilisées comprenant un complexe polyélectrolytique, un antigène et un polyol, **caractérisées en ce que** ledit complexe polyélectrolytique est un complexe de sulfate de dextrane et de poly-L-arginine, et ledit polyol est du mannitol, et dans lesquelles le mannitol est sous forme amorphe.

2. Les microsphères lyophilisées selon la revendication 1, dans lesquelles le complexe polyélectrolytique, le mannitol amorphe et l'antigène sont répartis de manière homogène.

3. Les microsphères lyophilisées selon les revendications 1 ou 2, dans lesquelles le mannitol amorphe, le complexe polyélectrolytique et l'antigène sont présents dans un rapport (pds/pds/pds, respectivement) de plages d'environ 1000/100/1 à environ 1/100/1000.

4. Une suspension comprenant les microsphères lyophilisées selon l'une quelconque des revendications 1 à 3.

5. La suspension selon la revendication 4, ladite suspension ayant des propriétés de double libération de médicament.

6. Une composition pharmaceutique comprenant les microsphères lyophilisées selon l'une quelconque des revendications 1 à 3 et un support pharmaceutiquement acceptable, la composition pharmaceutique comprenant une quantité pharmaceutiquement efficace de l'antigène.

7. La composition pharmaceutique selon la revendication 6, ladite composition pharmaceutique étant administrée via la voie parentérale.

8. La composition pharmaceutique selon l'une quelconque des revendications 6 à 7, pour utilisation dans l'administration de médicament extracellulaire.

9. La composition pharmaceutique selon l'une quelconque des revendications 6 à 7, pour utilisation dans l'administration d'un ou plusieurs médicaments à des phagocytes.

10. La composition pharmaceutique selon l'une quelconque des revendications 6 à 7, pour utilisation dans le traitement de maladies du système immunitaire, de maladies infectieuses, de maladies métaboliques et du cancer.

11. Un procédé pour la préparation de microsphères lyophilisées selon l'une quelconque des revendications 1 à 3, ledit procédé comprenant :
a) la préparation d'une charge aqueuse du complexe polyélectrolytique, de l'antigène et du polyol telle que définie dans la revendication 1 à une concentration en solides totale d'environ 0,1 à environ 10%,
b) le séchage par pulvérisation de la charge aqueuse préparée à l'étape a).
